# EUROPÄISCHE PATENTSCHRIFT

(11) **EP 1 534 431 B1**
(45) Veröffentlichungstag und Bekanntmachung des Hinweises auf die Patenterteilung: **25.02.2009**
(21) Anmeldenummer: 03773609.7
(22) Anmeldetag: 18.08.2003
(51) Int. Cl.: B01L 3/00, B29C 45/16

(54) **EINWEGKASSETTE**
DISPOSABLE CASSETTE
CASSETTE A USAGE UNIQUE

(30) Priorität: 28.08.2002 DE 10239597
(43) Veröffentlichungstag der Anmeldung: 01.06.2005
(73) Patentinhaber: Fresenius Medical Care Deutschland GmbH, 61352 Bad Homburg v.d.H. (DE)
(72) Erfinder: HÄCKER, Jürgen, 61267 Neu-Anspach (DE); FREY, Stephan, 60433 Frankfurt (DE); LAPP, Uwe, 35510 Butzbach (DE); JAHN, Paul, 60437 Frankfurt (DE); OESTERREICH, Stephan, 61267 Neu-Anspach (DE)
(74) Vertreter: Laufhütte, Dieter
(86) Internationale Anmeldenummer: PCT/EP2003/009128
(87) Internationale Veröffentlichungsnummer: WO 2004/024326

(56) Entgegenhaltungen:
- WO-A-02/24320
- WO-A-97/22825
- DE-A- 3 800 036
- US-A- 4 585 623
- US-A- 5 580 523
- JAROSCHEK C ET AL: "HARTE UND WEICHE KUNSTSTOFFE BEIM SPRITZGIESSEN KOMBINIEREN" KUNSTSTOFFE, CARL HANSER VERLAG. MUNCHEN, DE, Bd. 84, Nr. 6, 1. Juni 1994 (1994-06-01), Seiten 705-706,708, XP000445627 ISSN: 0023-5563
- STEINBICHLER G: "LSR IM VERBUND" KUNSTSTOFFBERATER, KUNSTSTOFF VERLAG. ISERNHAGEN, DE, Bd. 44, Nr. 5, Mai 1999 (1999-05), Seiten 28-30, XP000832868 ISSN: 0172-6374
- FREYER C ET AL: "MIT SILICONELASTOMEREN IM VERBUND" KUNSTSTOFFBERATER, KUNSTSTOFF VERLAG. ISERNHAGEN, DE, Bd. 7/8, Juli 2000 (2000-07), Seiten 27-30, XP000989450 ISSN: 0172-6374
- HUNOLD D ET AL: "SCHLAU KOMBINIERT - WIRTSCHAFTLICH GEFERTIGT MEHRKOMPONENTEN-SPRITZGIESSEN VON THERMO- UND DUROPLASTEN" KUNSTSTOFFE, CARL HANSER VERLAG. MUNCHEN, DE, Bd. 91, Nr. 3, März 2001 (2001-03), Seiten 108-110, XP001039129 ISSN: 0023-5563

## Beschreibung

Die Erfindung betrifft eine Einwegkassette nach dem Oberbegriff des Anspruchs 1.

In medizinischen Geräten kommen häufig Kunststoffeinmalartikel mit fluidführenden Kanälen zum Einsatz. Als Alternative zu herkömmlichen Schlauchsystemen haben sich hier entsprechende Kassettensysteme bewährt. In diesem Kassettensystem sind die entsprechenden Fluidwege gebildet. Auf das durch die Fluidwege fließende Fluid wird mittels entsprechender Aktoren eingewirkt. So sind beispielsweise Ventile eingesetzt, über die die Fluidwege durchgeschaltet oder verschlossen werden. Zum anderen sind in derartigen Kassettensystemen Pumpen zur Förderung des Fluids integriert. Im Bereich der medizinischen Anwendung sind bereits Einmalkassettensysteme bekannt, bei denen ein starrer Teil vorgesehen ist, in welchem Kanäle und Kammern eingelassen sind. Dieser starre Teil wird durch eine durchgehend flexible Folie abgedeckt.

Im Bereich der Analysentechnik sind die bekannten Kassettensysteme häufig recht kompliziert aufgebaut. Sie weisen einerseits starre Begrenzungen und andererseits Bereiche zum Einbau von Aktorelementen auf. Üblicherweise sind die in der Analysentechnik eingesetzten Systeme dreischichtig aufgebaut, indem neben zwei starren Schichten noch eine flexible Folie vorgesehen ist, durch die an freiliegenden Bereichen der Fluidfluß manipuliert werden kann.

Aus der WO 02/24320A1 ist bereits eine gattungsgemäße Einwegkassette zum Einsatz in der Analysentechnik bekannt, die aus einem ersten Teil besteht, in welchem in der Oberfläche Kanalstrukturen ausgenommen sind. Dieses erste Teil ist über ein zweites Teil dichtend abgedeckt. Eines der beiden Teile ist als flexibles Teil ausgebildet. An vorbestimmten Stellen dieser Einwegkassette sind Eingriffsbereiche für Aktorelemente vorgesehen. Das Ausbilden eines der beiden Teile der vorbekannten Einwegkassette als flexibles Teil ist allerdings von Nachteil, da die Stabilität wie auch die Funktionalität der einzelnen Dichtfunktion der gesamten der Einwegkassette nicht immer gewährleistet ist. Eine weitere Einwegkassette zeigt WO 97/22825 A1.

Aufgabe der vorliegenden Erfindung ist es daher, eine gattungsgemäße Einwegkassette derart weiterzubilden, daß sie einerseits möglichst einfach und zum anderen in sich stabil aufgebaut ist.

Erfindungsgemäß wird diese Aufgabe durch eine Einwegkassette mit den Merkmalen des Anspruchs 1 gelöst. Demnach sind das erste Teil und/oder das zweite Teil zum größten Teil starr ausgebildet. Es können jedoch jeweils flexibel ausgebildete Bereiche vorgesehen sein, wobei die starren und flexiblen Bereiche jeweils unter Verwendung der Zweikomponentenspritzgußtechnologie einstückig hergestellt sind. Dadurch kann das starre Teil mit den integrierten flexiblen Bereichen als ein Bauteil und somit in einem einzigen Produktionsschritt kostengünstig hergestellt werden. Zum anderen ergibt sich hier eine verglichen zum Stand der Technik hohe Stabilität des gesamten Bauteils. Aufgrund der weniger benötigten Schichten gegenüber anderen komplexer aufgebauten Einmalkassetten in der Analysentechnik ergibt sich auch eine kompaktere Bauform.

Vorteilhafte Ausgestaltungen der Erfindung sind in den Unteransprüchen wiedergegeben.

Demnach können die flexiblen Bereiche in den Eingriffsbereichen für die Aktorelemente ausgebildet sein.

Andererseits kann zumindest ein Teil der Kanäle aus flexiblem Material bestehen.

Als Aktoren können Ventile, Membranpumpen, Drosseln oder Dosierventile zum Einsatz kommen.

Der Bereich der Kanäle, in welchen die Aktoren eingekoppelt sind, sind in vorteilhafter Weise flacher und mit größerem Kanalquerschnitt ausgebildet. Durch diese Ausgestaltungen können die benötigten Aktorkräfte zum Auslenken des flexiblen Bereichs reduziert werden.

Als Fluid zur Durchströmung der Kanäle kann es sich sowohl um Flüssigkeiten als auch um Gase handeln.

Die erfindungsgemäße Kassette findet vorzugsweise in der Medizintechnik bei der Förderung und/oder Dosierung von Fluiden Anwendung. Besonders geeignet ist sie zur Förderung und/oder Dosierung in der Analysentechnik.

Weitere Einzelheiten und Vorteile der Erfindung werden anhand von in der Zeichnung dargestellten Ausführungsbeispielen näher erläutert. Es zeigen:
- Figur 1:: eine schematische perspektivische Detailansicht einer teilweise geschnittenen Einwegkassette nach einer Ausführungsvariante der vorliegenden Erfindung,
- Figur 2:: eine schematische Darstellung der Anordnung verschiedener Aktoren entlang eines Kanals in einer Einwegkassette gemäß Figur 1 und

- Figur 3:: eine perspektivische Darstellung einer weiteren Ausführungsvariante der erfindungsgemäßen Einwegkassette als herausgeschnittenes Detail.

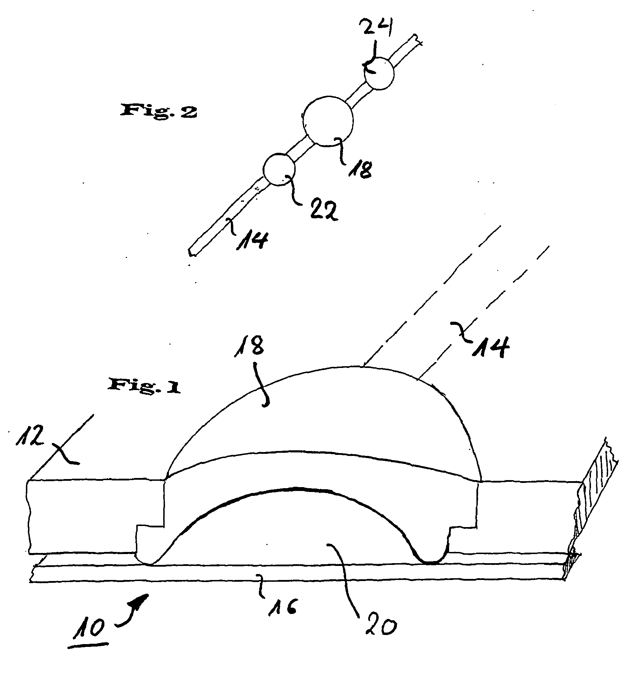

In der in Figur 1 teilweise dargestellten Einwegkassette 10 ist zum Zwecke der Fluidführung in einem ersten Teil 12 eine Kanalstruktur 14 ausgebildet, welche durch ein zweites Teil 16, einer Art starren Abdeckplatte, dicht abgeschlossen ist. Ein Teilbereich 18 des ansonsten aus einem starren Kunststoffmaterial bestehenden ersten Teils 12 ist als integraler Bestandteil des Teils 12 in einem flexiblen Elastomermaterial ausgeführt. Dieser einstückig ausgeführte Elastomerbereich 18 dient als Pumpkalotte. Durch eine Beaufschlagung mittels eines entsprechenden Aktuators, der in der Figur 1 hier nicht näher dargestellt ist, wird die Pumpkalotte hin- und herbewegt, so daß der sich von der Pumpkalotte 18 dichtend umschlossene Raum 20 in seinem Volumen verkleinert bzw. vergrößert. Dieser Raum 20, der mit dem Kanal 14 in Verbindung steht, ist mit dem zu fördernden Fluid gefüllt, so daß durch eine entsprechende Auslenkung der Elastomerkomponente 18, d.h. der Pumpkalotte, das Fluid entsprechend gefördert werden kann. Dieses Förderprinzip entspricht exakt demjenigen einer Membranpumpe und ist sowohl in der Analysentechnik wie insgesamt in der Medizintechnik als Prinzip bereits bekannt. In Figur 2 ist angedeutet, daß im Kanal 14 neben dem flexiblen Bereich 18, der als Pumpkalotte dient, ein- und ausgangsseitig Ventile 22 bzw. 24 vorgesehen sind, die als Rückstromsperre angebracht sind, die wechselseitig im Pumptakt geschlossen bzw. geöffnet werden. Auch diese Bereiche können als entsprechend flexible Teilbereiche des ansonsten starren ersten Teils 12 der Einmalkassette 10 ausgebildet sein. Das erste Teil 12 läßt sich unter Verwendung der Zweikomponentenspritzgußtechnologie als einstückiges Element produzieren.

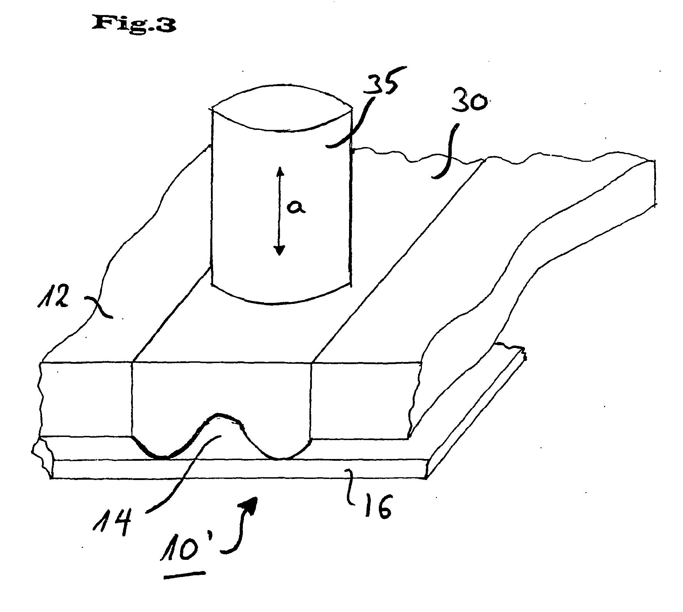

In Figur 3 ist eine andere Ausführungsvariante einer Einwegkassette 10' realisiert. Zum Zweck der Fluidführung ist einem ersten Teil 12 der Einwegkassette 10' eine Kanalstruktur 14 ausgebildet, welche durch das zweite Teil 16, welches als starre Abdeckplatte ausgebildet ist, nach unten dicht abgeschlossen ist. Der Kanal 14 läßt sich in Flußrichtung durch einen Kanalabschnitt 30 öffnen und verschließen, der in einem flexiblen Elastomermaterial ausgeführt ist und einstückig mit dem ersten Teil 12 durch Zweikomponentenspritzgußtechnik hergestellt wurde. Aufgrund dieser einstückigen Ausbildung ist der elastische Bereich 30 integraler Bestandteil des ansonsten starren ersten Teils 12. Dadurch ist er zum einen fest und zum anderen flüssigkeitsdicht mit diesem verbunden.

Zum Schließen eines derartigen Ventils reicht es aus, die Kraft eines Stößels 35 in Doppelpfeilrichtung a auf die Rückseite des elastischen Bereichs 30 zu leiten. Der Kanal 14 wird aufgrund dieser Krafteinleitung verschlossen. Das Öffnen des Ventils erfolgt durch Wegnahme der Schließkraft, d.h das Zurückziehen des Stößels 35 in Doppelpfeilrichtung a, so daß sich der Bereich 30 durch seine elastischen Materialeigenschaften wieder in die ursprüngliche Kanalform zurück verformt.

Im hier dargestellten Ausführungsbeispiel ist der Kanal 14 durchgehend als flexibles Elastomerteil ausgeführt. Alternativ dazu kann aber der Kanal nur im Bereich des Ventils selbst als flexibler elastomerer Bereich ausgeführt sein, während die übrigen Kanalbereiche mittels des starren Materials des ersten Teils 12 gebildet sind. Die Krafteinleitung auf den flexiblen Elastomerbereich kann auf verschiedene Weise erfolgen. Einerseits taktil über einen Stößel 35, wie in Figur 3 dargestellt. Andererseits aber auch direkt pneumatisch über Druckluft. In diesem Fall wäre eine Dichtlippe rund um das Ventil auf der Rückseite des ersten Teils 12 vorzusehen. Schließlich kann die Kraft auch indirekt pneumatisch über hydraulisch über ein kleines Druckkissen aufgebracht werden, welches geräteseitig vorzusehen wäre. Besonders vorteilhaft ist es hier, daß schon mit sehr geringen Ventilschließkräften eine Dichtfunktion erreicht werden kann.

## Patentansprüche

1. Einwegkassette bestehend aus zumindest einem ersten Teil, in welchen in der Oberfläche Kanalstrukturen ausgenommen sind, und einem dieses dichtend abdeckenden zweiten Teil, wobei an vorbestimmten Stellen Eingriffsbereiche für Aktorelemente vorgesehen sind,
**dadurch gekennzeichnet,**
**daß** das erste Teil zum größten Teil starr ausgebildet ist, wobei es jedoch in den Eingriffsbereichen für die Aktorelemente flexibel ausgebildete Bereiche aufweist und/oder das zweite Teil zum größten Teil starr ausgebildet ist, wobei es jedoch in den Eingriffsbereichen für die Aktorelemente flexibel ausgebildete Bereiche aufweist, und
wobei die starren und flexiblen Bereiche unter Verwendung der Zweikomponentenspritzgußtechnologie einstückig hergestellt sind.

2. Einwegkassette nach Anspruch 1, **dadurch gekennzeichnet, daß** zumindest ein Teil der Kanäle aus flexiblem Material besteht.

3. Einwegkassette nach einem der Ansprüche 1 bis 2, **dadurch gekennzeichnet, daß** als Aktoren Ventile zum Einsatz kommen.

4. Einwegkassette nach einem der Ansprüche 1 bis 2, **dadurch gekennzeichnet, daß** als Aktoren Membranpumpen zum Einsatz kommen.

5. Einwegkassette nach einem der Ansprüche 1 bis 2, **dadurch gekennzeichnet, daß** als Aktoren Drosseln oder Dosierventile zum Einsatz kommen.

6. Einwegkassette nach einem der Ansprüche 1 bis 5, **dadurch gekennzeichnet, daß** der Bereich der Kanäle, in welchem die Aktoren eingekoppelt sind, flacher und mit größerem Kanalquerschnitt ausgebildet sind.

## Claims

1. A disposable cassette comprising at least a first part in which passage structures are cut out in the surface and a second part sealingly covering the first part, wherein engagement regions for actuator elements are provided at predetermined locations,
**characterised in that**
the first part is for the major part rigid, wherein however it has flexible regions in the engagement regions for the actuator elements and/or the second part is for the major part rigid, wherein however it has flexible regions in the engagement regions for the actuator elements, and
wherein the rigid and flexible regions are produced in one piece using two-component injection moulding technology.

2. A disposable cassette according to claim 1 **characterised in that** at least some of the passages comprise flexible material.

3. A disposable cassette according to one of claims 1 and 2 **characterised in that** valves are used as actuators.

4. A disposable cassette according to one of claims 1 and 2 **characterised in that** diaphragm pumps are used as actuators.

5. A disposable cassette according to one of claims 1 and 2 **characterised in that** throttles or metering valves are used as actuators.

6. A disposable cassette according to one of claims 1 to 5 **characterised in that** the region of the passages in which the actuators are coupled are shallower and of larger passage cross-section.

## Revendications

1. Cassette à usage unique constituée d'au moins une première partie, dans laquelle sont évidées dans la surface des structures de canal, et une deuxième partie recouvrant d'une manière étanche celle-ci, où sont prévues à des emplacements prédéterminés des zones d'engagement pour des éléments acteurs,
**caractérisée en ce que**
la première partie est réalisée majoritairement d'une manière rigide, en présentant cependant dans les zones d'engagement pour les éléments acteurs des zones réalisées d'une manière flexible et/ou la deuxième partie est réalisée majoritairement d'une manière rigide, en présentant cependant dans les zones d'engagement pour les éléments acteurs des zones réalisées d'une manière flexible, et
où les zones rigides et flexibles, en utilisant la technologie de moulage par injection à deux composants, sont réalisées en une pièce.

2. Cassette à usage unique selon la revendication 1, **caractérisée en ce qu'**au moins une partie des canaux est réalisée en matériau flexible.

3. Cassette à usage unique selon l'une des revendications 1 à 2, **caractérisée en ce que** des vannes sont utilisées comme acteurs.

4. Cassette à usage unique selon l'une des revendications 1 à 2, **caractérisée en ce que** des pompes à membrane sont utilisées comme acteurs.

5. Cassette à usage unique selon l'une des revendications 1 à 2, **caractérisée en ce que** des papillons ou vannes de dosage sont utilisés comme acteurs.

6. Cassette à usage unique selon l'une des revendications 1 à 5, **caractérisée en ce que** la zone des canaux, dans laquelle sont couplés les acteurs, sont réalisées plus plates et avec une section transversale de canal plus grande.
